# EUROPÄISCHE PATENTSCHRIFT

(11) **EP 1 084 705 B1**
(45) Veröffentlichungstag und Bekanntmachung des Hinweises auf die Patenterteilung: **25.06.2014**
(21) Anmeldenummer: 00119496.8
(22) Anmeldetag: 24.04.1997
(51) Int. Cl.: A61K 31/00, A61K 31/401, A61P 3/10, A61K 31/40, A61K 31/426

(54) **Verfahren zur Senkung des Blutglukosespiegels in Säugern**
Method for lowering blood glucose levels in mammals
Méthode pour diminuer le taux de glucose sanguin des mammifères

(30) Priorität: 25.04.1996 DE 19616486
(43) Veröffentlichungstag der Anmeldung: 21.03.2001
(62) Teilanmeldung aus: 97924866.3
(73) Patentinhaber: Royalty Pharma Collection Trust, Wilmington DE 19890 (US)
(72) Erfinder: Demuth, Hans-Ulrich, 06114 Halle (DE); Rosche, Fred, 06184 Dieskau (DE); Schmidt, Jörn, 06114 Halle (DE); Pauly, Robert P., Dr., Walter McKenzie Centre, Edmonton, AB T6G 2B7 (CA); McIntosh, Christopher H.S., Vancouver, B.C. V6T 1T7 (CA); Pederson, Ray A., Vancouver, B.C. V6S 1K9 (CA)
(74) Vertreter: Bühler, Dirk

(56) Entgegenhaltungen:
- EP-A- 0 708 179
- WO-A-91/17767
- MENTLEIN ROLF ET AL: "Dipeptidyl-peptidase IV hydrolyses gastric inhibitory polypeptide, glucagon-like peptide-1(7-36)-amide, peptide histidine methionine and is responsible for their degradation in human serum." EUROPEAN JOURNAL OF BIOCHEMISTRY, Bd. 214, Nr. 3, 1993, Seiten 829-835, XP000921372 ISSN: 0014-2956
- ENDROCZI, E. ET AL: "Dipeptidyl peptidase IV (DP IV) and superoxide dismutase activity in thymus-derived lymphocytes: effects of inhibitory peptides and zinc in vitro" ACTA PHYSIOL. HUNG. (1990), 75(1), 35-44 , XP008000941
- ASHWORTH, DOREEN M. ET AL: "2-Cyanopyrrolidides as potent, stable inhibitors of dipeptidyl peptidase IV" BIOORG. MED. CHEM. LETT. (1996), 6(10), 1163-1166 , XP000957893

## Beschreibung

Die Erfindung betrifft ein einfaches Verfahren zur Senkung der Blutzuckerkonzentration mit Hilfe von aktivitätsmindernden *Effektoren* (Substraten, Pseudosubstraten, Inhibitoren, Bindungsproteinen, Antikörpern u. a.) entsprechend Anspruch 1.

Neben Proteasen, die in unspezifische Proteolyse einbezogen sind, was letztlich den Abbau von Proteinen zu Aminosäuren bewirkt, kennt man regulatorische Proteasen, die an der Funktionalisierung (Aktivierung, Deaktivierung, Modulierung) von endogenen Peptidwirkstoffen beteiligt sind [KIRSCHKE, H., LANGNER, J., RIEMANN, S., WIEDERANDERS, B., ANSORGE, S. und BOHLEY, P., Lysosomal cysteine proteases. Excerpta Medica (Ciba Foundation Symposium 75), 15 (1980); KRÄUSSLICH, H.-G. and WIMMER, E., Viral Proteinases. Ann. Rev. Biochem. 57, 701 (1987)]. Insbesondere im Zusammenhang mit der Immunforschung und der Neuropeptidforschung sind eine Reihe solcher sogenannten Konvertasen, Signalpeptidasen oder Enkephalinasen entdeckt worden [GOMEZ, S., GLUSCHANKOF, P., LEPAGE, A., MARRAKCHI, N. and COHEN, P., Proc. Natl. Acad. Sci. USA 85, 5468 (1988); ANSORGE, S. and SCHÖNE, E., Histochem. 82, 41 (1987)].

Aufgrund der Häufigkeit des Vorkommens der Aminosäure Prolin in einer Vielzahl von Peptidhormonen und den damit verbundenen Struktureigenschaften dieser Peptide wird für prolinspezifische Peptidasen eine den Signalpeptidasen analoge Funktion diskutiert [YARON, A., The Role of Proline in the Proteolytic Regulation of Biologically Active Peptides. Biopolymers 26, 215 (1987); WALTER, R., SIMMONS, W.H. and YOSHIMOTO, T., Proline Specific Endo- and Exopeptidases. Mol. Cell. Biochem. 30, 111 (1980); VANHOOF, G., GOOSSENS, F., DE MEESTER, I., HENDRIKS, D. and SCHARPE, S., Proline motifs and their biological processing. FASEB Journal 9, 736 (1995)]. Dabei bestimmt Prolin in diesen Peptiden durch seine besondere Struktur sowohl Konformation als auch Stabilität dieser Peptide, indem sie vor Abbau durch unspezifische Proteasen schützt [KESSLER, H., Konformation und biologische Wirkung von zyklischen Peptiden. Angew. Chem. 94, 509 (1982)]. Enzyme, die dagegen hochspezifisch strukturverändernd auf Prolin-haltige Sequenzen einwirken (HIV-Protease, Cyclophylin u. a.) sind attraktive Ziele der aktuellen Wirkstoff-Forschung. Insbesondere für die nach dem Prolin spaltenden Peptidasen Prolyl Endopeptidase (PEP) und Dipeptidyl Peptidase IV (DP IV) konnten Beziehungen zwischen der Modulation der biologischen Aktivität von natürlichen Peptidsubstraten und deren selektiver Spaltung durch diese Enzyme wahrscheinlich gemacht werden. So nimmt man an, daß PEP eine Rolle beim Lernen bzw. im Gedächtnisprozeß spielt und DP IV in die Signalübertragung während der Immunantwort einbezogen ist [ISHIURA, S., TSUKAHARA, T., TABIRA, T., SHIMIZU, T., ARAHATA K. and SUGITA, H., FEBS-Letters 260, 131 (1990); HEGEN, M., NIEDOBI-TEK, G., KLEIN, C.E., STEIN, H. and FLEISCHER, B., J. of Immunology 144, 2908 (1990)].

Ähnlich wie die außerordentliche Prolinspezifität dieser Enzyme wird ihre hohe Selektivität für die Aminosaure Alanin innerhalb typischer Erkennungsregionen in Substraten dieser Enzyme diskutiert, wonach Alanin-haltige Peptide ähnliche Konformationen einnehmen können wie strukturanaloge Prolin-haltige Peptide. Kürzlich wurden derartige Eigenschaften Alaninhaltiger Peptidketten durch Punktmutation (Austausch von Prolin gegen Alanin) nachgewiesen [DODGE, R.W. and SCHERAGA, H.A., Folding and unfolding kinetics of the proline-toalanine mutants of bovine pancreatic ribonuclease A. Biochemistry 35 (5) 1548 (1996)].

DP IV- bzw. DP IV-analoge Aktivität (z. B. besitzt die cytosolische DP II eine der DP IV nahezu identische Substratspezifität) kommt im Blutkreislauf vor, wo sie hochspezifisch Dipeptide vom N-Terminus biologisch aktiver Peptide abspaltet, wenn Prolin oder Alanin die benachbarten Reste der N-terminalen Aminosäure in deren Sequenz darstellen. Deshalb wird davon ausgegangen, daß dieses Enzym an der Regulation von Polypeptiden *in vivo* beteiligt ist [VANHOOF, G., GOOSSENS, F., DE MEESTER, I., HENDRIKS, D. and SCHARPE, S., Proline motifs and their biological processing, FASEB Journal 9, 736 (1995)].

Die Glukose-abhängigen insulinotropen Polypeptide: Gastric Inhibitory Polypeptide 1-42 (GIP₁₋₄₂) und Glucagon-Like Peptide Amide-1 7-36 (GLP-1₇₋₃₆). Hormone, die die Glukoseinduzierte Insulinsekretion des Pankreas stimulieren (auch *Incretine*), sind Substrate der DP IV, da sie von den N-terminalen Sequenzen dieser Peptide die Dipeptide Tyrosinyl-Alanin bzw. Histidyl-Alanin *in vitro* und *in situ* abspalten kann [MENTLEIN, R., GALLWITZ, B., and SCHMIDT, W.E., Dipeptidyl Peptidase IV hydrolyzes gastric inhibitory polypeptide, glucagon-like peptide-1(7-36)amide, peptide histidine methionine and is responsible for their degradation in human serum. Eur. J. Biochem. 214, 829 (1993)].

Die Reduktion derartiger DP IV- bzw. DP IV-analoger Enzymaktivität zur Spaltung solcher Substrate *in vivo* kann dazu dienen unerwünschte Enzymaktivität unter Laborbedingungen als auch in pathologischen Zuständen von Säuger-Organismen wirksam zu unterdrücken [DEMUTH, H.-U., Recent developments in the irreversible inhibition of serine and cysteine proteases.J. Enyzeme Inhibition 3, 249-278 (1990); DEMUTH, H.-U. and HEINS, J., On the catalytic Mechanism of Dipeptidyl Peptidase IV. in Dipeptidyl Peptidase IV (CD 26) in Meta-bolism and the Immune Response (B. Fleischer, Ed.) R.G. Landes, Biomedical Publishers, Georgetown, 1-35 (1995)]. Z. B. basiert *Diabetes mellitus* Typ II (auch Altersdiabetes) auf einer verminderten Insulinsekretion bzw. Störungen in der Rezeptorfunktion, die u. a. in proteolytisch bedingten Konzentrationsanomalien der Incretine begründet sind [BROWN, J.C., DAHL, M., KWAWK, S., MCINTOSH, C.H.S., OTTE, S.C. and PEDERSON, R.A. Peptides 2, 241 (1981); SCHMIDT, W.E., SIEGEL, E.G., GALLWITZ, B. KUMMEL, H., EBERT, R. and CREUTZFELDT, W., Characterization of the inulinotropic activity of fragments derived from gastric inhibitory polypeptide. Diabetologia 29, 591A (1986); ADELHORST, K., HE-DEGAARD, B.B., KNUDSEN, L.B. and KIRK, O., Structure-activity studies of glucagon-like peptide. J. Biol. Chem. 296, 6275 (1994)].

Hyperglykämie und damit verbundene Ursachen bzw. Folgeerscheinungen (auch *Diabetes mellitus*)werden nach gegenwärtigem Stand der Technik durch die Verabreichung von Insulin (z.B. von aus Rinderpankreas isoliertem oder auch gentechnisch gewonnenem Material) an erkrankte Organismen in verschiedenen Darreichungsformen behandelt. Alle bisher bekannten, als auch die moderneren Verfahren zeichnen sich durch hohen Materialaufwand, hohe Kosten und oft durch entscheidende Beeinträchtigungen der Lebensqualität der Patienten aus. Die klassische Methode (tägliche *i*.*v*.. Insulin-Injektion, üblich seit den dreißiger Jahren) behandelt die akuten Krankheitssymptome, führt aber nach längerer Anwendung u. a. zu schweren Gefäßveränderungen (Arteriosklerose) und Nervenschädigungen [LACY, P., Status of Islet Cell Transplantation. Diabetes Care 16 (3) 76 (1993)].

Neuerdings wird die Installation subkutaner Depot-Implantate (die Insulinabgabe erfolgt dosiert, und die täglichen Injektionen entfallen) sowie die Implantation (Transplantation) intakter Langerhansscher Zellen in die funktionsgestörte Pankreasdrüse oder andere Organe und Gewebe vorgeschlagen. Derartige Transplantationen sind technisch aufwendig. Weiterhin stellen sie einen risikobehafteten chirurgischen Eingriff in den Empfängerorganismus dar und verlangen auch bei Zellverpflanzungen nach Methoden zur Suppression bzw. der Umgehung des Immunsystems [LACY, P., Treating Diabetes with Transplanted Cells. Sci. Americ. 273 (1)40-46(1995)].

Die möglichst orale Applikation hochaffiner, niedermolekularer Enzyminhibitoren dagegen ist eine kostengünstigere Alternative z. B. zu invasiven chirurgischen Techniken bei der Behandlung pathologischer Erscheinungen. Derartige Enzyminhibitoren finden inzwischen therapeutischen Einsatz als Immunsuppressiva, Antithrombotika und als AIDS-Virostatika. Durch chemisches Design von Stabilitäts-, Transport- und Clearence-Eigenschaften kann deren Wirkungsweise modifiziert und auf individuelle Eigenschaften abgestimmt werden [SANDLER, M. and SMITH, H.J., Hrsg., Design of Enzyme Inhibitors as Drugs. Oxford University Press, Oxford (1989); MUNRÖ, J.E., SHEPHERD, T.A., JUNGHEIM, L.N., HORNBACK, W.J., HATCH, S.D., MÜSING, M.A., WISKERCHEN, M.A., SU, K.S., CAMPANALE, K.M., BAXTER, A.J., and COLACINO, J.M., Potent, orally bioavailable HIV-1 protease inhibitors containing noncoded D-amino acids. Bioorg. Medicinal Chem. Letters 5 (23) 2897 (1995)].

Das Ziel der Erfindung ist ein einfaches und neuartiges Verfahren zur Senkung des Blutglukosespiegels, das erfindungsgemäß dadurch erreicht werden kann, daß mittels Verabreichung von aktivitätsmindernden Effektoren an einen Säugerorganismus, in kausaler Folge die endogenen (oder zusätzlich exogen verabreichten) insulinotropen Peptide GIP₁₋₄₂ und GLP-1₇₋₃₆ durch DP IV vermindert abgebaut werden und damit die Konzentrationsabnahme dieser Pepdidhormone verringert bzw. verzögert wird, bei der Behandlung von Diabetes mellitus.

Der Erfindung liegt der überraschende Befund zugrunde, daß eine Reduktion der im Blutkreislauf agierenden DP IV- Aktivität kausal zur Beeinflussung des Blutzuckerspiegels führt. Es wurde gefunden, daß
1. die Verminderung von DP IV- Aktivität zu relativer Stabilitätserhöhung der Glukose-stimulierten, oder extern zugeführten Incretine zur Folge hat. d.h. durch Applikation von aktivitätsmindernden Effektoren der DP IV der Incretin-Abbau im Blut kontrolliert werden kann.
2. erhöhte biologische Abbaustabilität der Incretine eine Wirkungsveränderung endogenen Insulins zur Folge hat.
3. Die durch Reduktion der DP IV- Aktivität im Blut erzielte Stabilitätserhöhung der Incretine in nachfolgender Veränderung der Glukoseinduzierten Insulinwirkung resultiert und damit zu einer mittels aktivitätsmindernden DP IV- Effektoren kontrollierbaren Modulierung des BlutGlukosespiegels führt.

Die Erfindung betrifft somit aktivitätsmindernde Effektoren der Dipeptidyl Peptidase IV (DP IV)- Enzymaktivität zur Verwendung zur Senkung des Blutzuckerspiegels unter die für Hyperglykämie charakteristische Glukosekonzentration im Serum eines Säuger-Organismus zur Milderung von Diabetes mellitus.

Die erfindungsgemäß applizierten aktivitätsmindernden Effektoren der DP IV können in pharmazeutisch anwendbaren Formulierungskomplexen als Inhibitoren, Substrate, Pseudosubstrate, Inhibitoren der DP IV-Expression, Bindungsproteine oder Antikörper dieser Enzymproteine oder Kombinationen aus diesen verschiedenen Stoffen, die DP IV- Proteinkonzentration im Säugerorganismus reduzieren, zum Einsatz kommen. Erfindungsgemäße aktivitätsmindernde Effektoren sind z.B. DP IV-Inhibitoren wie die Dipeptidderivate bzw. Dipeptidmimetika Alanyl-Pyrolidid, Isoleucyl-Thiazolidid sowie das Pseudosubstrat N-Valyl-Prolyl, O-Benzoyl Hydroxylamin. Derartige Verbindungen sind aus der Literatur bekannt [DEMUTH, H.-U., Recent developments in the irreversible inhibition of serine and cysteine proteases. J. Enzyme Inhibition 3, 249 (1990)] oder in Analogie zu den in der Literatur beschriebenen Methoden herstellbar.

Das offenbarte Verfahren stellt eine neuartige Herangehensweise zur Senkung erhöh-ter Blutglukosekonzentration im Serum von Säugern dar. Es ist einfach, kommerziell nutzbar und zur Anwendung bei der Therapie, insbesondere von Erkrankungen, die auf überdurch-schnittlichen Blutglukosewerten basieren, in der Humanmedizin geeignet.

Die aktivitätsmindernden Effektoren werden in Form von pharmazeutischen Präparaten enthaltend den Wirkstoff in Kombination mit üblichen aus dem Stand der Technik bekannten Trägermaterialien verabreicht. Beispielsweise werden sie parenteral (z.B. *i*.*v*. in physiologischer Kochsalzlösung) oder enteral (z.B. oral, formuliert mit üblichen Trägermaterialien wie z. B. Glukose) appli-ziert.

In Abhängigkeit von ihrer endogenen Stabilität und ihrer Bioverfügbarkeit müßen einfache oder auch mehrfache Gaben der aktivitätsmindernden Effektoren erfolgen, um die erwünschte Normalisierung der Blutglukosewerte zu erreichen. Z. B. kann im Falle von Aminoacyl-Thiazolididen ein solcher Dosisbereich zwischen 1.0 mg und 10.0 mg Effektorsubstanz pro Kilogramm liegen.

### Abbildugen:

Abb. 1: MALDI-TOF-Analyse der DP IV-katalysierten Hydrolyse von GIP₁₋₄₂ (b) und GLP₇₋₃₆ und deren Hemmung durch Isoleucyl-Thiazolidid (a).
Abb. 2: HPLC-Analyse der Serumpräsenz von GLP-1 Metaboliten in Gegenwart und in Abwesenheit DP IV Inhibitors Isoleucyl-Thiazolidid *in vivo.*
Abb. 3: Einfluss des DP IV-Inhibitors Isoleucyl-Thiazolidid auf verschiedene Blutparameter der *i.d.*-Glukose-stimulierten Ratte.

### Ausführungsbeispiele

### Beispiel 1: Inhibierung der DP IV-katalysierten Hydrolyse der Incretine GIP₁₋₄₂ und GLP-1₇₋₃₆ in situ

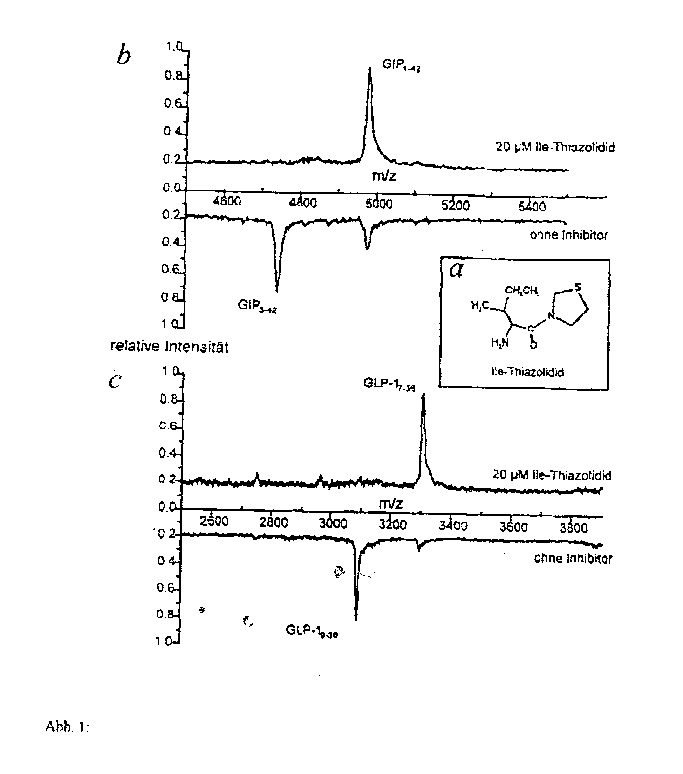

Sowohl *in vitro* mit gereinigtem Enyzm als auch *in situ*, z.B. in gepooltem humanem Serum, kann man die Hydrolyse der Incretine, verursacht durch DP IV- Aktivität, nachweisen bzw. mit Hilfe von Inhibitoren unterdrücken (Abb. 1).

Erfindungsgemäß erreicht man *in situ* bei Inkubation von 30 µM GIP₁₋₄₂ bzw. 30 µM GLP-1₇₋₃₆ und 20 µM Isoleucyl-Thiazolidid (1a), einem reversiblen DP IV-Inhibitor in 20 %-igem Serum bei pH 7.6 und 30 °C die komplette Unterdrückung der Enzym-katalysierten Hydrolyse beider Peptid-hormone innerhalb von 24 Stunden (1b und 1c, jeweils obere Spektren. Synthetisches GIP₁₋₄₂ (5 µM) und synthetisches GLP-1₇₋₃₆ (15 µM) wurden mit humanem Serum (20 %) in 0.1 mM TRICINE Puffer bei pH 7.6 und 30 °C für 24 Stunden inkubiert. Proben der Inkubationsansätze (für GIP₁₋₄₂ 2.5 pmol und im Falle von GLP-1₇₋₃₆ 7.5 pmol) wurden nach verschiedenen Zeiten entnommen. Die Proben wurden mit 2',6'-Dihydroxyacetophenon als Matrix co-kristallisiert und mittels MALDI-TOF-Massen-spektrometrie analysiert. Die Spektren (Abb. 1) stellen Akkumulationen von 250 einzelnen Laserschüssen pro Probe dar.
(1b) Die Signale im Bereich von *m*/*z* 4980.1 ± 5.3 entsprechen GIP₁₋₄₂ (*M* 4975.6) und *m*/*z* 4745.2 ± 5.5 dem DP IV-Hydrolyseprodukt GIP₃₋₄₂ (*M*4740.4).
(1c) Die Signale *m*/*z* 3325.0 ± 1.2 entsprechen GLP-1₇₋₃₆ (M 3297.7) und *m*/*z* 3116.7 ± 1.3 dem DP IV-Hydrolyseprodukt GLP-1₉₋₃₆ (*M* 3089.6).

In den Versuchsansätzen ohne Inhibitor wurden die Incretine in dieser Zeit fast vollständig abgebaut (Abb. 1b und 1c, jeweils untere Spektren).

### Beispiel 2: Inhibierung des Abbaus von GLP-1₇₋₃₆ durch den DP IV-Inhibitor Isoleucyl-Thiazolidid in vivo.

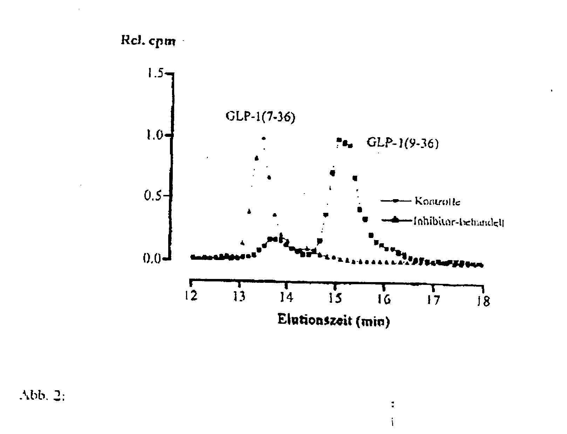

Verfolgt man den Metabolismus der nativen Incretine (hier GLP-1₇₋₃₆) im Serum der Ratte in Abhängigkeit in Gegenwart des DP IV-Inhibitors Isoleucyl-Thiazolidid (*i*.*v*. Injektion einer 1.5 µM Inhibitorlösung in 0.9 % -iger Kochsalzlösung) gegenüber einer Kontrolle, so ist bei einer Konzentration des Inhibitors Isoleucyl-Thiazolidid von ca. 0.1 mg/kg Laborratte bei den Inhibitor-behandelten Versuchstieren (n = 5) im Verlaufe des Versuchszeitraums kein Abbau des insulino-tropen Peptidhormons GLP-1₇₋₃₆ zu beobachten (Abb. 2).

Zur Detektion der Metaboliten in Anwesenheit und Abwesenheit des DP IV-Inhibitors (20 Minuten nach vorheriger *i*.*v*.-Inhibitor- bzw Kochsalzgabe) erhielten die Versuchs- und Kontrolltiere *i.v*. 50 - 100 pM ¹²⁵I-GLP-1₇₋₃₆ (spezifische Aktivität ca. 1 µMCi/pM). Blutproben wurden nach 2 - 5 min entnommen und das Plasma mittels 20 % Acetonitril extrahiert. Nachfolgend wurde der Peptidextrakt mittels RP-HPLC separiert und die Radioaktivität der Fraktionen an einem γ-Counter analysiert. Die gefundene Aktivität ist in cpm (*counts per minute* relativ zum Maximum angegeben).

### Beispiel 3: Modulation der Insulinwirkung und Senkung des Blutglukosespiegels nach i.v. Applikation des DP IV-Inhibitors Isoleuzyl-Thiazolidid in vivo.

An der durch intraduodenale (*i*.*d*.) Injektion Glukose-stimulierten Ratte, kann durch *i*.*v*. Gabe verschiedener DP IV-Effektoren, z. B. von 0.1 mg Isoleucyl-Thiazolidid pro kg Ratte eine auf die Inhibitorwirkung zurückgehende, zeitlich verzögert einsetzende Senkung des Glukosespiegels beobachtet werden. Dieser Effekt ist dosisabhängig und nach Absetzen der Infusion von 0.05 mg/min des DP IV-Inhibitors Isoleucyl-Thiazolidid pro kg Ratte reversibel. Die *i*.*v*. Applikation der gleichen Glukosemenge von Inhibitor-behandelten und Kontroll-Tieren zeigt im Gegensatz zur den *i*.*d*. Glukose-stimulierten Versuchstieren keine vergleichbare Wirkung.

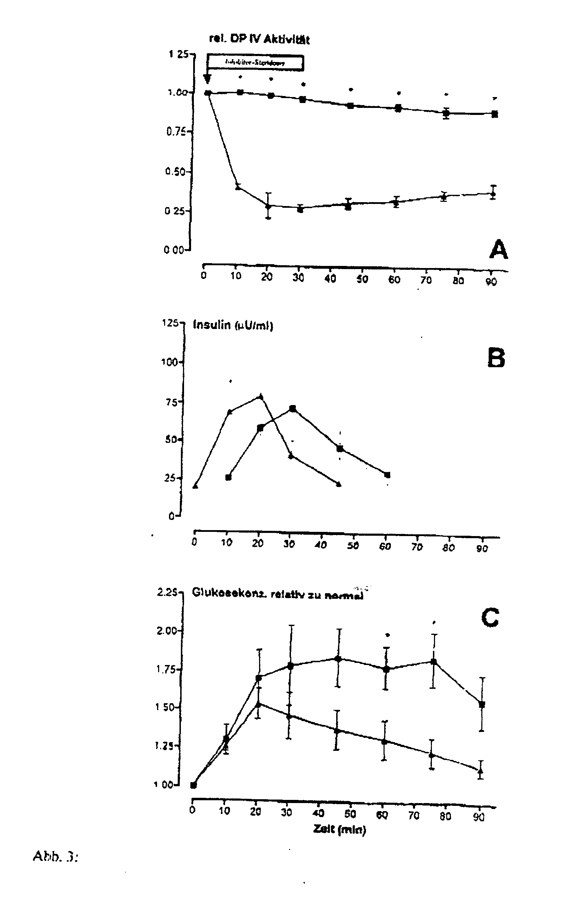

Abbildung 3 verdeutlicht diese Zusammenhänge an den Inhibitor-abhängigen Veränderungen der Plasmaparameter: A - DP IV-Aktivität, B - Plasma-Insulinspiegel, C - Blutglukosespiegel.

Die Versuchstiere (n = 5, männliche Wistar-Ratten, 200 - 225 g) erhielten als Initialdosis 1.5 µM Isoleucyl-Thiazolidid in 0.9 % -iger Kochsalzlösung (A) oder gleiche Volumina 0.9 % - ige Kochsalzlösung ohne Inhibitor (■) (Kontrollgruppe n = 5). Die Versuchsgruppe erhielt weiterhin eine Infusion des Inhibitors von 0.75 SM/min über 30 min Versuchszeit (*). Der Kontrollgruppe wurde im gleichen Zeitraum eine Inhibitor-freie 0.9 % -ige Kochsalzlösung infundiert. Zum Zeitpunkt t=0 erhielten die Tiere *i*.*d*. eine Glukosedosis von 1g/kg 40 % -iger Dextroselösung (w/v).

Allen Versuchstieren wurden Blutproben in zehn Minutenabständen entnommen.

Glukose Messungen erfolgten am Vollblut (Lifescan One Touch II analyzer) während die DP IV-Aktivität und die Insulinkonzentrationen im Plasma bestimmt wurden.

Der hier angewandte Insulintest ist empfindlich zwischen 10 und 160 mU/ml [PEDERSON, R.A., BUCHAN, A.M.J., ZAHEDI-ASH, S., CHEN, C.B. and BROWN, J.C. Reg. Peptides. 3, 53-63 (1982)]. Die DP IV-Aktivität wurde spektralphotometrisch bestimmt [DEMUTH, H.-U. and HEINS, J., On the catalytic Mechanism of Dipeptidyl Peptidase IV. in Dipeptidyl Peptidase IV (CD 26) in Metabolism and the Immune Response (B. Fleischer, Ed.) R.G. Landes, Biomedical Publishers, Georgetown, 1-35 (1995)]. Alle Messwerte sind als Mittelwerte mit Standardabweichung angegeben.

## Patentansprüche

1. Aktivitätsmindernder Effektor der Dipeptidylpeptidase IV (DP IV)-Enzymaktivität zur Verwendung zur Senkung des Blutzuckerspiegels unter die für Hyperglykämie charakteristische Glukosekonzentation im Serum eines Säuger-Organismus zur Milderung von Diabetes mellitus, wobei besagter Effektor zum verminderten Abbau der endogenen insulinotropen Peptide GIP₁₋₄₂ und GLP-1₇₋₃₆ durch DP IV führt.

2. Aktivitätsmindernder Effektor zur Verwendung gemäß Anspruch 1, wobei besagter Effektor ein DP IV-Inhibitor ist.

3. Aktivitätsmindernder Effektor zur Verwendung gemäß Anspruch 1 oder 2, wobei besagter Effektor parenteral oder enteral appliziert wird.

4. Aktivitätsmindernder Effektor zur Verwendung gemäß Anspruch 3, wobei besagter Effektor mit üblichen Trägermateralien formuliert oral appliziert wird.

## Claims

1. Activity-lowering effector of dipeptidylpeptidase IV (DP IV)-enzymatic activity for use in lowering the blood glucose level below the glucose concentration in the serum of a mammalian organism characteristic of hyperglycemia for alleviation of diabetes mellitus, wherein said effector results in the reduced degradation of the endogenous insulinotropic peptides GIP₁₋₄₂ and GLP-1₇₋₃₆ by DP IV.

2. Activity-lowering effector for use according to claim 1, wherein said effector is a DP IV-inhibitor.

3. Activity-lowering effector for use according to claim 1 or 2, wherein said effector is applied parenterally or enterally.

4. Activity-lowering effector for use according to claim 3, wherein said effector is applied orally together with usual carrier materials.

## Revendications

1. Effecteur réduisant l'activité de l'enzyme dipeptidylpeptidase IV (DP IV) pour l'utilisation pour abaisser le taux de sucre dans le sang en dessous de la concentration en glucose caractéristique de l'hyperglycémie dans le sérum d'un organisme de mammifère pour soulager le diabète sucré, dans lequel ledit effecteur conduit à une dégradation réduite des peptides endogènes insulinotropes GIP₁₋₄₂ et GLP-1₇₋₃₆ par la DP IV.

2. Effecteur réducteur d'activité pour l'utilisation selon la revendication 1, dans lequel ledit effecteur est un inhibiteur de DP IV.

3. Effecteur réducteur d'activité pour l'utilisation selon la revendication 1 ou 2, dans lequel ledit effecteur est appliqué par voie parentérale ou entérale.

4. Effecteur réducteur d'activité pour l'utilisation selon la revendication 3, dans lequel ledit effecteur est appliqué par voie orale avec les supports usuels.
